**Europäisches Patentamt**

**European Patent Office**

**Office européen des brevets**

(11) Numéro de publication : **0 350 344 B1**

(12)

# FASCICULE DE BREVET EUROPEEN

(45) Date de publication du fascicule du brevet :
**09.12.92 Bulletin 92/50**

(51) Int. Cl.⁵ : **C01B 13/14**

(21) Numéro de dépôt : **89401515.5**

(22) Date de dépôt : **02.06.89**

(54) **Oxydes métalliques à structure lamellaire pontée et procédé de fabrication.**

(30) Priorité : **10.06.88 FR 8807748**

(43) Date de publication de la demande :
**10.01.90 Bulletin 90/02**

(45) Mention de la délivrance du brevet :
**09.12.92 Bulletin 92/50**

(84) Etats contractants désignés :
**AT BE CH DE ES FR GB GR IT LI LU NL SE**

(56) Documents cités :
**WO-A-88/00093
REVUE DE CHIMIE MINERALE, vol. 19, nos.
4-5, 1982, pages 485-495; P. ALDEBERT et al.:
"V2O5 gels: a versatile host structurefor intercalation"
IND. ENG. CHEM. PRODUCT RESEARCH AND
DEVELOPMENT, vol. 22, no. 4, décembre 1983,
pages 553-559, American Chemical Society,Washington, US; M.L. OCCELLI: "Catalytic
cracking with an interlayered clay. A twodimensional molecular sieve"**

(73) Titulaire : **RHONE POULENC CHIMIE
25 Quai Paul Doumer
F-92408 Courbevoie Cédex (FR)**

(72) Inventeur : **Kairy, Mostafa
1, rue Bossuet Appartement 641
F-45100 Orléans (FR)**
Inventeur : **Tinet, Daniel
12, rue Antoine Macarel
F-45100 Orléans (FR)**
Inventeur : **Van Damme, Henri
166, Allée des Seringas
F-45160 Olivet (FR)**

(74) Mandataire : **Esson, Jean-Pierre et al
RHONE-POULENC CHIMIE Service Brevets
Chimie 25, quai Paul Doumer
F-92408 Courbevoie Cedex (FR)**

EP 0 350 344 B1

**Description**

La présente invention concerne un procédé de fabrication d'oxydes métalliques à structure lamellaire pontée et oxydes métalliques obtenus.

Elle se rapporte plus particulièrement à un procédé de pontage d'oxydes métalliques à structure lamellaire pour obtenir un oxyde métallique présentant une surface spécifique élevée.

De nombreux travaux sont effectués pour fabriquer des produits présentant une surface spécifique élevée, notamment utiles en adsorption, absorption ou catalyse. Les zéolites représentent actuellement les produits les plus utilisés dans ces applications. Toutefois, des produits de substitution à ces zéolites sont activement recherchés. En effet, les zéolites présentent des inconvénients, notamment une limitation de la taille des canaux.

Parmi ces produits de substitution, les composés à structure lamellaire pontée sont très intéressants. Ainsi, on connaît déjà de nombreuses argiles pontées avec des pontages de différentes natures.

Par pontage, on entend l'intercalation d'espèces chimiques ioniques ou non entre les feuillets de la structure lamellaire d'un produit, provoquant un gonflement du produit par écartement des différents plans, puis la fixation par liaison physique ou chimique de ces espèces sur les feuillets de la structure lamellaire.

Dans les cas où les espèces intercalées ne sons pas fixées, le phénomène est réversible et est appelé intercalation.

L'intercalation et le pontage de différents oxydes métalliques à structure lamellaire ont également été étudiés. Ainsi, les gels de pentoxyde de vanadium présentent une structure lamellaire et sont constitués de particules colloïdales ayant la forme de rubans pouvant s'empiler les uns sur les autres, qui joue le rôle d'hôte pour de nombreux composés chimiques tels que des solvants organiques, des cations à longues chaînes ou des métallocènes, comme décrit dans les articles de J. LIVAGE etal.

Le nombre d'espèces intercalables et notamment de cations dépend de la charge et des nombreux sites chargés présents sur chaque feuillet ou plan de la structure lamellaire. Dans le cas des argiles, la charge est due à la substitution des cations de silicium et/ou d'aluminium par des cations de charge inférieure. Dans les gels de pentoxyde de vanadium, la charge peut être obtenue par la réduction des cations de vanadium. L'intercalation met alors en jeu un transfert d'électrons, conjugué à l'insertion de l'espèce à intercaler dans le réseau. Toutefois, cette insertion peut modifier la structure ou détruire le réseau à partir d'un certain taux de réduction.

Il n'a pas été possible, jusqu'à présent, d'intercaler dans les oxydes métalliques du type pentoxyde de vanadium, des espèces ioniques volumineuses fortement chargées, comme le cation $[Al_{13}O_4(OH)_{24}]^{7+}$ permettant notamment d'obtenir des propriétés catalytiques importantes.

Un des buts de la présente invention est de remédier notamment à ces inconvénients en proposant un procédé permettant d'intercaler des espèces ioniques globulaires fortement chargées dans des oxydes métalliques à structure lamellaire, sans modifier ou détruire la structure de l'oxyde.

A cet effet, l'invention propose un procédé pour la fabrication d'oxyde métallique à structure lamellaire pontée consistant à mélanger un gel d'oxyde métallique à structure lamellaire avec une solution de cations inorganiques possédant un rapport e/r supérieur à 2 environ, e étant la charge électronique du cation et r le rayon ionique en Angströms et/ou de cations organiques de diamètre moléculaire supérieur à 6 Å, puis à ajouter au milieu réactionnel une solution contenant une espèce ionique globulaire, à laisser reposer ce mélange pendant un certain temps pour que l'échange entre les cations précités et les espèces ioniques globulaires se réalise. Enfin, l'oxyde métallique ponté est récupéré par tout moyen tel que filtration.

Selon une caractéristique de l'invention, les gels d'oxyde métallique lamellaire sont choisis dans le groupe comprenant les gels de pentoxyde de vanadium, d'oxyde de molybdène et d'oxyde de tungstène ou d'oxyde de titane.

Par espèce ionique globulaire, on entend des cations complexes de grand rayon ionique tels que, par exemple, les polyoxocations métalliques d'aluminium, zirconium, chrome, molybdène, niobium ou tantale, comme $[Al_{13}O_4(OH)_{24}]^{7+}$ ; $[Zr_4(OH)_{14}(H_2O)_{10}]^{4+}$ ; $[Mo_6Cl_{14}]^{4+}$ ; $[Nb_6Cl_{12}]^{2+}$ ; $[Ta_6Cl_{12}]^{2+}$.

L'espèce ionique globulaire préférée de l'invention est le polyoxocation d'aluminium, et notamment le polyoxocation $[Al_{13}O_4(OH)_{24}]^{7+}$.

Les cations inorganiques de rapport e/r supérieur à 2 convenables pour l'invention sont notamment ceux du groupe comprenant les cations d'alcalino-terreux, de cobalt, fer, manganèse, cuivre, strontium, lithium, d'oxyde de vanadyle ($VO^{2+}$), d'aluminium, de nickel, des lanthanides et plus particulièrement de lanthane.

Comme cations organiques de diamètre moléculaire supérieur à 6 Å convenables à l'invention, on peut citer les cations d'ammonium quaternaire ou de phosphonium quaternaire comprenant des radicaux organiques aliphatiques substitués ou non et comportant de 3 à 20 atomes de carbone tels que, par exemple, le cation de tétrabutyl d'ammonium et de tétrapropyl d'ammonium.

2

Avantageusement, quand l'oxyde métallique est l'oxyde de vanadium ($V_2O_5$), le cation préféré est le cation $VO^{2+}$. Ce cation est intercalé dans la structure lamellaire par échange avec un de ces sels tels que, par exemple, le sulfate $VOSO_4$.

Selon une nouvelle caractéristique de l'invention, la solution des espèces ioniques globulaires a un pH inférieur à 5 environ quand l'oxyde métallique est l'oxyde de vanadium ($V_2O_5$).

En effet, le gel d'oxyde de vanadium n'est pas stable à des pH supérieurs.

De préférence, le pH de la solution d'espèces ioniques globulaires est compris entre 3 environ et 4,5 environ.

Le rapport molaire $OH^-/Al$ dans la solution de l'espèce ionique globulaire est compris entre 0 et 2,5, de préférence entre 1,2 et 2,2.

Les solutions de cations ou d'espèces ioniques globulaires sont généralement des solutions aqueuses, toutefois il est possible d'utiliser d'autres solvants tels que, par exemple, le méthanol, l'éthanol.

Les gels d'oxyde métallique sont obtenus selon des procédés classiques, comme par exemple par précipitation d'un sel précurseur de cet oxyde métallique.

L'oxyde métallique préféré de l'invention est le pentoxyde de vanadium, utilisé sous forme de gel à une concentration en $V_2O_5$ comprise entre 0,1 et 1 % environ en poids d'oxyde métallique.

Il est également préféré comme espèce ionique globulaire le polyoxocation d'aluminium qui peut être représenté par la formule $[Al_{13}O_4(OH)_{24}]^{7+}$. Cette espèce ionique globulaire est ajoutée de manière à avoir dans le milieu réactionnel contenant le gel de $V_2O_5$ une quantité d'aluminium comprise entre 13 mmole environ et 50 mmole environ par gramme de $V_2O_5$. Cette concentration est exprimée en mole de Al.

Le mélange du gel d'oxyde métallique et de la solution d'espèce ionique globulaire est laissé mûrir de préférence pendant 1 heure environ à 100 heures environ. Toutefois, la durée de mûrissement est fonction de la température. Généralement, le mûrissement de la solution est conduit à une température comprise entre la température ambiante et 70°C environ.

Après le mûrissement de la solution, l'oxyde métallique est extrait du milieu réactionnel, par exemple par filtration.

Le solide ainsi récupéré peut être séché à l'air et éventuellement calciné avant son utilisation comme adsorbant ou catalyseur.

Selon un procédé préféré de l'invention, le solide filtré est d'abord lavé pour notamment éliminer les cations tels que les cations $VO^{2+}$ intercalés dans la première étape du procédé.

Ce lavage peut être effectué de manière classique par de l'eau. Toutefois, dans un mode de réalisation préféré de l'invention, ce lavage est réalisé par dialyse.

L'invention a également pour objet un oxyde de vanadium à structure lamellaire pontée dont la distance entre chaque plan ou hauteur de pilier est comprise entre 8 Å et 13 Å.

Cet oxyde de vanadium présente une surface spécifique mesurée par la méthode B.E.T. supérieure à 20 $m^2/g$. Cette surface spécifique est dans le mode de réalisation préféré de l'invention comprise entre 50 environ et 100 $m^2/g$ environ.

L'oxyde de vanadium de l'invention a une surface spécifique stable et comprise dans les valeurs indiquées ci-dessus, dans un large domaine de température et notamment jusqu'à 350°C environ.

Dans le mode de réalisation préféré de l'invention, l'oxyde de vanadium contient, exprimé en poids d'aluminium, de 15 à 35 % en poids environ de polyoxocation d'aluminium, et de préférence entre 25 et 35 % en poids.

Les composés de l'invention peuvent être utilisés dans le domaine de la filtration par tamisage moléculaire, ou comme catalyseur ou support de catalyseur.

L'invention sera plus clairement illustrée au vu des exemples donnés ci-dessous uniquement à titre indicatif.

Exemples 1 à 6

PREPARATION D'UN GEL DE PENTOXYDE DE VANADIUM

Un gel de pentoxyde de vanadium est préparé par passage d'une solution de métavanadate de sodium sur une résine échangeuse d'ions H+. La concentration en métavanadate de sodium est de 1 mole/litre.

La solution obtenue est une solution d'acide décanavanadique qui polymérise spontanément.

Après quelques heures de vieillissement, la solution se transforme en un gel rouge sombre visqueux de formule générale $V_2O_5nH_2O$, à une concentration pondérale en $V_2O_5$ égale à 10 à 20 g/l.

D'autres procédés de préparation de gel de $V_2O_5$ peuvent être utilisés tels qu'une trempe à l'eau de $V_2O_5$ fondu à 720°C, décrit par P. ALDEBERT et al, dans J.Coll - Inter.Sci, 98, 478 (1984), ou par hypertrempe sur rouleau refroidi à la température de l'azote liquide, décrit dans L. RIVOALEN et al, J. NON. Cr Solids, 21, 171 (1976).

Enfin, une autre méthode, décrite par E. RUIZ et al dans J. Chem. Soc. Faraday - Trans, 1, 82, 1597-1604 (1986), permet d'obtenir un gel de $V_2O_5$ convenable pour l'invention. Des gels de $V_2O_5$ peuvent être également obtenus par précipitation à partir de précurseurs organiques.

## PREPARATION DE LA SOLUTION D'ESPECES IONIQUES GLOBULAIRES D'ALUMINIUM

La solution de polyoxocation d'aluminium est préparée par addition d'une solution de soude 0,2 M dans une solution de nitrate d'aluminium à une concentration comprise entre 0,1 M et 0,2 M.

Le rapport $OH^-/Al$ dans la solution est compris entre 0,5 et 2,5 et le pH est compris entre 2,5 et 5.

De préférence, on laisse mûrir la solution, ce qui favorisera l'intercalation de ce polyoxocation dans la structure du gel $V_2O_5$.

Le polyoxocation d'aluminium a été décrit notamment par JOHANSSON dans Acta. Chem. Scandinavian, 1960, 14 n° 3.

Ainsi, JOHANSSON a étudié la structure de ce cation qui comprend douze atomes d'aluminium octaédriques, entourant un aluminium tétraédrique. Le rayon ionique de ce cation à l'état hydraté est de 12,5 Å et 5,4 Å à l'état non hydraté.

Le rapport $OH^-/Al$ a une influence sur la stabilité de la solution, et il est préférable de travailler avec un rapport voisin de 2.

Il est également préférable de purifier cette solution avant son mélange avec le gel d'oxyde métallique. Cette purification peut être réalisée par dialyse.

## PONTAGE DU GEL DE $V_2O_5$

On ajoute au gel de $V_2O_5$ préparé ci-dessus, après dilution, jusqu'à une concentration pondérale égale à 0,5 % en $V_2O_5$, une solution de sulfate de vanadyl ($VOSO_4, 5H_2O$) à une concentration $10^{-2}$N.

Le mélange est agité fortement et l'on observe la formation d'un précipité verdâtre dû à l'intercalation d'ions vanadyl $VO^{++}$ avec deux couches de molécule d'eau.

Le produit obtenu, isolé et séché, présente une surface spécifique faible, de l'ordre de 3 m2/g.

Toutefois, pour intercaler le cation $[Al_{13}O_4(OH)_{24}]^{7+}$, le produit obtenu précédemment n'est avantageusement pas isolé du milieu réactionnel, la solution du cation d'aluminium préparée étant ajoutée directement dans ce milieu.

Le mélange obtenu est alors maintenu sous agitation pendant environ 1 heure, puis laissé mûrir pendant au moins 48 heures, à température ambiante.

Après ce mûrissement, le produit est récupéré, par exemple par filtration ou centrifugation puis lavé avec de l'eau distillée.

Dans cet exemple, le produit est ensuite mis en suspension dans de l'eau et dialysé.

Le produit ainsi obtenu est séché par chauffage en étuve ou en lit fluidisé à l'air, à température ambiante.

## CARACTERISATION DU PRODUIT PONTE

Les produits présentent une porosité comprise entre 0,1 cm3/g et 0,4 cm³/g mesurée par la méthode BET. Cette porosité est stable jusqu'à 250°C et varie peu jusqu 'à 350°C.

Les diamètres des pores s'étalent entre 0,1 et 200 Å environ.

Des analyses par les méthodes RPE, Spectrographie Infrarouge et diffraction des rayons X montrent que le pontage n'est pas effectué entre tous les feuillets de la structure lamellaire.

Dans le tableau I ci-dessous, sont résumés les caractéristiques des oxydes de vanadium pontés obtenus avec différents taux de pontage ou à des conditions opératoires différentes.

A titre de comparaison, il est également indiqué les caractéristiques d'un oxyde de vanadium non ponté.

| Essai | Solution de polyoxocations d'aluminium pH | Solution de polyoxocations d'aluminium R = OH⁻/Al | Durée mûrissement (H) | Rapport Al/V$_2$O$_5$ meq de Al dans mélange | Rapport en poids (%) Al/V dans V$_2$O$_5$ ponté | Distance entre plans (Å) | Surface spécifique m2/g |
|---|---|---|---|---|---|---|---|
| 1 (V$_2$O$_5$ non ponté) | - | - | - | 0 | 0 | 0 | 2 à 3 |
| 2 | 3,92 | 1,6 | 24 | 45 | 25 | 8 | 57 |
| 3 | 4,20 | 2,3 | 24 | 45 | 29 | 8-13 | 90 |
| 4 | 4,15 | 2,5 | 24 | 45 | 30 | 8-13 | 64 |
| 5 | 4,00 | 2 | 24 | 80 | 32 | 8-13 | 90 |
| 6 | 3,95 | 2 | 48 | 120 | 32 | 8-13 | 97 |

5

Exemples 7 - 8

PREPARATION DE SOLUTIONS D'ESPECES IONIQUES GLOBULAIRES

-Espèce ionique globulaire de chlorure polynucléaire de Molybdène

5 grammes de $MoCl_2$ sont dissous dans 1 litre de méthanol déshydraté. Après trois mois, la solution est filtrée pour éliminer la fraction non dissoute. La solution obtenue est jaune. Le solide $Mo_6Cl_{14}$ est récupéré par évaporation du solvant sous vide.

-Espèce ionique globulaire de chlorure polynucléaire de Niobium

Un mélange de Nb $Cl_5$ et NaCl est broyé et placé dans un tube avec du niobium métallique. Ce tube est chauffé sous vide à 850°C pendant 6 à 8 heures et maintenu à cette température pendant 12 heures.

Après refroidissement, le produit est extrait par extractions successives avec 2 l d'eau contenant 1 ml de HCl concentré.

La solution est filtrée puis traitée avec un volume égal d'acide chlorhydrique concentré. Le mélange est alors chauffé et agité jusqu'à précipitation complète de $Nb_6$ $Cl_{14}$ $8H_2O$.

Ces deux espèces ioniques ont comme formule $Mo_6$ $Cl_{12}^{2+}$ et $Nb_6$ $Cl_{12}^{2+}$. Toutefois, il est également indiqué dans la littérature que ces espèces ioniques peuvent avoir trois charges positives.

PONTAGE D'UN GEL DE $V_2O_5$

- Exemple 7

On ajoute au gel de $V_2O_5$ contenant comme espèces intercalées des cations de vanadyl $VO^{++}$, préparé selon le mode opératoire décrit à l'exemple 1, une solution éthanolique de chlorure polynucléaire de Molybdène contenant au moins 1,6 millimole de ce complexe par litre de solution.

Après agitation de la solution, celle-ci est maintenue pendant 24 heures à 60°C.

Le résidu obtenu est centrifugé. Le culot de centrifugation est ensuite dispersé dans 50 ml d'eau.

La suspension obtenue est dialysée puis lyophilisée.

Le produit obtenu a une surface spécifique de 15 m²/g et une distance entre plans de 6 Å.

- Exemple 8

On répète le mode opératoire indiqué ci-dessus, à l'exception que la solution de chlorure polynucléaire de molybdène est remplacée par une solution de chlorure polynucléaire de niobium contenant au moins 1,6 millimole de complexe par litre de solution.

Le produit obtenu a une surface spécifique de 18 m²/g environ et une distance entre plans de 5,2 Å.

Ainsi l'invention permet d'obtenir un oxyde métallique comme le pentoxyde de vanadium présentant une surface spécifique élevée stable sur des plages étendues de températures et des propriétés catalytiques améliorées.

Ces composés peuvent être utilisés comme catalyseur dans de nombreuses réactions, notamment de synthèse organique, requérant un catalyseur à caractère acide.

A titre d'exemple, la catalyse de l'hydroxylation du phénol par $H_2O_2$ sera décrite ci-dessous :

Dans un réacteur, préalablement purgé avec de l'azote, on charge 9,4 g de phénol et 0,25 g de catalyseur fabriqué selon l'exemple 3 ci-dessus. L'ensemble est chauffé à 80°C sous agitation.

Une solution d'eau oxygénée à 70 % en poids par volume est injectée dans le mélange.

Après 2 h 30 de réaction, le milieu réactionnel est filtré et la teneur en $H_2O_2$ est déterminée par iodométrie tandis que les diphénols formés pyrocatéchine, hydroquinone sont déterminés par analyse chromatographique en phase liquide.

Les résultats obtenus sont :

```
-  taux de transformation de H₂O₂        99,5 %
-  rendement en pyrocatéchine par         7,5 %
   rapport à H₂O₂ transformée
-  rendement en hydroquinone par          8   %
   rapport à H₂O₂ transformée
-  rendement total en diphénols          15,5 %
```

## Revendications

1. Procédé pour la fabrication d'oxyde métallique à structure lamellaire pontée, caractérisé en ce qu'il consiste :
   - à mélanger un gel d'un oxyde métallique à structure lamellaire avec une solution de cations inorganiques possédant un rapport e/r supérieur à 1,5, e étant la charge du cation et r le rayon ionique et/ou un cation organique de diamètre moléculaire supérieur à 6 Å,
   - puis à ajouter au milieu réactionnel une solution contenant une espèce ionique globulaire,
   - à laisser ce mélange reposer,
   - à filtrer la solution, et à sécher le solide recueilli.

2. Procédé selon la revendication 1, caractérisé en ce que le solide recueilli par filtration est lavé avant séchage.

3. Procédé selon la revendication 1 ou 2, caractérisé en ce que l'oxyde métallique est choisi dans le groupe comprenant l'oxyde de vanadium, l'oxyde de molybdène, l'oxyde de tungstène, l'oxyde de titane.

4. Procédé selon l'une des revendications précédentes, caractérisé en ce que l'espèce ionique globulaire est un polyoxocation d'aluminium, de chrome, zirconium, nickel, molybdène, niobium, tantale.

5. Procédé selon la revendication 4, caractérisé en ce que l'espèce ionique globulaire est choisie dans le groupe comprenant les cations $[Al_{13}O_4(OH)_{24}]^{7+}$, $[Zr_4(OH)_{12}(H_2O)_{10}]^{4+}$, $[Mo_6Cl_{14}]^{4+}$, $[Nb_6Cl_{12}]^{2+}$, $[Ta_6Cl_{12}]^{2+}$.

6. Procédé selon l'une des revendications précédentes, caractérisé en ce que le cation inorganique est choisi dans le groupe comprenant les alcalino-terreux, les cations de cobalt, fer, manganèse, cuivre, strontium, lithium, de vanadyle, aluminium, nickel, lanthane et lanthanides.

7. Procédé selon l'une des revendications 1 à 5, caractérisé en ce que le cation organique précité est choisi dans le groupe des cations d'ammonium quaternaire ou de phosphonium quaternaire comportant des radicaux alkyles substitués ou non ayant de 3 à 20 atomes de carbone.

8. Procédé selon la revendication 7, caractérisé en ce que le cation organique précité est le cation de tétrabutyl d'ammonium, tétrapropyl d'ammonium.

9. Procédé selon l'une des revendications 2 à 8, caractérisé en ce que le lavage du solide recueilli comprend au moins une étape de dialyse.

10. Procédé selon l'une des revendications précédentes, caractérisé en ce que le solide recueilli est calciné après séchage.

11. Procédé selon l'une des revendications précédentes, caractérisé en ce que le gel d'oxyde métallique à structure lamellaire est un gel de pentoxyde de vanadium.

12. Procédé selon la revendication 11, caractérisé en ce que le gel d'oxyde de vanadium contient de 0,1 à 1 % en poids de $V_2O_5$.

13. Procédé selon la revendication 11 ou 12, caractérisé en ce que l'espèce ionique globulaire est le polyoxocation d'aluminium.

14. Procédé selon la revendication 13, caractérisé en ce que la solution de l'espèce ionique globulaire est laissée mûrir.

15. Procédé selon l'une des revendications 11 à 14, caractérisé en ce que le mélange du gel d'oxyde de vanadium avec la solution de l'espèce ionique contient de 13 à 50 mmole de Al par g de $V_2O_5$.

16. Oxyde de vanadium à structure lamellaire pontée caractérisé en ce qu'il a une distance entre plans ou une hauteur de pilier comprise entre 8 Å et 13 Å.

17. Oxyde de vanadium selon la revendication 16, caractérisé en ce qu'il a une surface spécifique stabilisée supérieure à 20 m$^2$/g, de préférence comprise entre 50 et 100 m$^2$/g.

18. Oxyde de vanadium selon l'une des revendications 16 ou 17, caractérisé en ce qu'il contient de 15 à 35 % en poids d'aluminium exprimé en Al, de préférence entre 25 et 35 % en poids d'aluminium.

19. Utilisation de l'oxyde de vanadium selon l'une des revendications 16 à 18, comme catalyseur des réactions de synthèse organique à catalyse acide.

20. Utilisation de l'oxyde de vanadium selon la revendication 19, caractérisée en ce que la réaction de synthèse organique est l'hydroxylation du phénol par l'eau oxygénée.


## Patentansprüche

1. Verfahren zur Herstellung eines Metalloxids mit lamellarer überbrückter Struktur, dadurch gekennzeichnet, daß man:
   - ein Gel eines Metalloxids mit lamellarer Struktur mit einer Lösung von anorganischen Kationen, die ein Verhältnis e/r größer 1,5 aufweisen, wobei e die Ladung des Kations und r der Ionenradius ist, und/oder eines organischen Kations mit einem Moleküldurchmesser größer 6 Å vermischt,
   - dem Reaktionsgemisch eine Lösung zusetzt, die ein kugeliges Ion enthält,
   - dieses Gemisch ruhen läßt und
   - die Lösung filtriert und den erhaltenen Feststoff trocknet.

2. Verfahren nach Anspruch 1, dadurch gekennzeichnet, daß der abfiltrierte Feststoff vor dem Trocknen gewaschen wird.

3. Verfahren nach Anspruch 1 oder 2, dadurch gekennzeichnet, daß das Metalloxid aus der Gruppe ausgewählt wird, die Vanadiumoxid, Molybdänoxid, Wolframoxid und Titanoxid umfaßt.

4. Verfahren nach einem der vorangehenden Ansprüche, dadurch gekennzeichnet, daß das kugelige Ion ein Polyoxokation von Aluminium, Chrom, Zirkonium, Nickel, Molybdän, Niob und Tantal ist.

5. Verfahren nach Anspruch 4, dadurch gekennzeichnet, daß das kugelige Ion ausgewählt wird aus der Gruppe, die die Kationen $[Al_{13}O_4(OH)_{24}]^{7+}$, $[Zr_4(OH)_{12}(H_2O)_{10}]^{4+}$, $[Mo_6Cl_{14}]^{4+}$, $[Nb_6Cl_{12}]^{2+}$, $[Ta_6Cl_{12}]^{2+}$ umfaßt.

6. Verfahren nach einem der vorangehenden Ansprüche, dadurch gekennzeichnet, daß das anorganische Kation aus der Gruppe ausgewählt wird, die die Erdalkali, die Kationen von Kobalt, Eisen, Mangan, Kupfer, Strontium, Lithium, Vanadyl, Aluminium, Nickel, Lanthan und Lanthaniden umfaßt.

7. Verfahren nach einem der Ansprüche 1 bis 5, dadurch gekennzeichnet, daß das vorgenannte organische Kation ausgewählt wird aus der Gruppe der quaternären Ammoniumkationen oder quaternären Phosphoniumkationen, die substituierte oder nicht substituierte Alkylreste mit 3 bis 20 Kohlenstoffatomen enthalten.

8. Verfahren nach Anspruch 7, dadurch gekennzeichnet, daß das genannte organische Kation das Tetrabutylammoniumkation oder das Tetrapropylammoniumkation ist.

9. Verfahren nach einem der Ansprüche 2 bis 8, dadurch gekennzeichnet, daß das Waschen des isolierten Feststoffes mindestens eine Dialysestufe umfaßt.

10. Verfahren nach einem der vorangehenden Ansprüche, dadurch gekennzeichnet, daß der isolierte Feststoff nach dem Trocknen gebrannt wird.

11. Verfahren nach einem der vorangehenden Ansprüche, dadurch gekennzeichnet, daß das Gel des Metalloxids mit lamellarer Struktur ein Vanadiumpentoxid-Gel ist.

12. Verfahren nach Anspruch 11, dadurch gekennzeichnet, daß das Vanadiumpentoxid-Gel 0,1 bis 1 Gew.-% $V_2O_5$ enthält.

13. Verfahren nach Anspruch 11 oder 12, dadurch gekennzeichnet, daß das kugelige Ion das Aluminiumpolyoxokation ist.

14. Verfahren nach Anspruch 13, dadurch gekennzeichnet, daß man die Lösung des kugeligen Ions reifen läßt.

15. Verfahren nach einem der Ansprüche 11 bis 14, dadurch gekennzeichnet, daß das Gemisch von Vanadiumoxid-Gel und Lösung des Ions 13 bis 50 mmol Al je g $V_2O_5$ enthält.

16. Vanadiumoxid mit verbrückter lamellarer Struktur, dadurch gekennzeichnet, daß es einen Abstand zwischen den Ebenen oder eine Pfeilerhöhe von 8 bis 13 Å aufweist.

17. Vanadiumoxid nach Anspruch 16, dadurch gekennzeichnet, daß es eine stabilisierte spezifische Oberfläche von mehr als 20 m²/g, vorzugsweise von 50 bis 100 m²/g, aufweist.

18. Vanadiumoxid nach einem der Ansprüche 16 oder 17, dadurch gekennzeichnet, daß es 15 bis 35 Gew.-% Aluminium, ausgedrückt als Al, vorzugsweise 25 bis 35 Gew.-% Aluminium, enthält.

19. Verwendung des Vanadiumoxids nach einem der Ansprüche 16 bis 18 als Katalysator für organische Synthesereaktionen mit saurer Katalyse.

20. Verwendung des Vanadiumoxids nach Anspruch 19, dadurch gekennzeichnet, daß die organische Synthesereaktion eine Hydroxylierung von Phenol mit Wasserstoffperoxid ist.


## Claims

1. Process for the manufacture of a metal oxide having a bridged lamellar structure, characterised in that it consists:
   - in mixing a gel of a metal oxide having a lamellar structure with a solution of inorganic cations possessing a ratio e/r of more than 1.5, e being the charge on the cation and r the ionic radius, and/or an organic cation having a molecular diameter of more than 6 Å,
   - then in adding a solution containing a globular ionic species to the reaction medium,
   - in leaving this mixture to stand,
   - in filtering the solution, and in drying the solid collected.

2. Process according to claim 1, characterised in that the solid collected by filtration is washed before being dried.

3. Process according to claim 1 or 2, characterised in that the metal oxide is chosen from the group comprising vanadium oxide, molybdenum oxide, tungsten oxide and titanium oxide.

4. Process according to one of the preceding claims, characterised in that the globular ionic species is a polyoxocation of aluminium, chromium, zirconium, nickel, molybdenum, niobium or tantalum.

5. Process according to claim 4, characterised in that the globular ionic species is chosen from the group comprising the cations $[Al_{13}O_4(OH)_{24}]^{7+}$; $[Zr_4(OH)_{12}(H_2O)_{10}]^{4+}$; $[Mo_6Cl_{14}]^{4+}$; $[Nb_6Cl_{12}]^{2+}$; $[Ta_6Cl_{12}]^{2+}$.

6. Process according to one of the preceding claims, characterised in that the inorganic cation is chosen from the group comprising alkaline earth metals and cobalt, iron, manganese, copper, strontium, lithium, vanadyl, aluminium, nickel, lanthanum and lanthanide cations.

7. Process according to one of claims 1 to 5, characterised in that the abovementioned organic cation is chosen from the group comprising quaternary ammonium or quaternary phosphonium cations containing substituted or unsubstituted alkyl radicals having from 3 to 20 carbon atoms.

8. Process according to claim 7, characterised in that the abovementioned organic cation is a tetrabutylammonium or tetrapropylammonium cation.

9. Process according to one of claims 2 to 8, characterised in that the washing of the solid collected comprises at least one dialysis stage.

10. Process according to one of the preceding claims, characterised in that the solid collected is calcined after being dried.

11. Process according to one of the preceding claims, characterised in that the metal oxide gel having a lamellar structure is a vanadium pentoxide gel.

12. Process according to claim 11, characterised in that the vanadium oxide gel contains from 0.1 to 1% by weight of $V_2O_5$.

13. Process according to claim 11 or 12, characterised in that the globular ionic species is the aluminium polyoxocation.

14. Process according to claim 13, characterised in that the solution of the globular ionic species is left to ripen.

15. Process according to one of claims 11 to 14, characterised in that the mixture of the vanadium oxide gel with the solution of the ionic species contains from 13 to 50 mmol of Al per g of $V_2O_5$.

16. Vanadium oxide having a bridged lamellar structure, characterised in that it has an inter-plane spacing or column height of between 8 Å and 13 Å.

17. Vanadium oxide according to claim 16, characterised in that it has a stabilised specific surface area of more than 20 m²/g, and preferably between 50 and 100 m²/g.

18. Vanadium oxide according to one of claims 16 and 17, characterised in that it contains from 15 to 35% by weight of aluminium expressed as Al, and preferably between 25 and 35% by weight of aluminium.

19. Use of the vanadium oxide according to one of claims 16 to 18 as a catalyst for acid-catalysed organic synthesis reactions.

20. Use of the vanadium oxide according to claim 19, characterised in that the organic synthesis reaction is the hydroxylation of phenol with hydrogen peroxide.